# EUROPEAN PATENT APPLICATION

(11) **EP 4 119 094 A1**
(43) Date of publication of application: **18.01.2023**
(21) Application number: 21768520.5
(22) Date of filing: 08.03.2021
(51) Int. Cl.: A61F 2/06, A61F 2/90

(54) **MEDICAL APPARATUS FOR DILATING TARGET PART**

(30) Priority: 09.03.2020 US 202062986811 P
(71) Applicant: Chen, Chieh-Hsiao, Taichung City, Taiwan 406 (CN)
(72) Inventor: Chen, Chieh-Hsiao, Taichung City, Taiwan 406 (CN)
(74) Representative: Lucke, Andreas
(86) International application number: PCT/CN2021/079583
(87) International publication number: WO 2021/180041

(57) **Abstract**

A medical device for dilating a target tissue includes: a first lateral portion; a second lateral portion; and a middle portion connecting the first lateral portion and the second lateral portion and disposed between the first lateral portion and the second lateral portion. The medical device has a dilate mechanism. The dilate mechanism allows the first lateral portion and the second lateral portion to be in a first dilated state when a first criterion is met, and allows the middle portion to be in a second dilated state when a second criterion is met, but does not allow the middle portion to be in the second dilated state when the first criterion is met.

## Description

### BACKGROUND OF THE INVENTION

### FIELD OF THE INVENTION

The present disclosure relates to a medical device for dilating a target tissue and, more particularly, to a medical device adapted to dilate a target tissue and having lateral portions dilated upon commencement of a dilating process.

### DESCRIPTION OF THE PRIOR ART

Existing conventional medical devices for dilating a target tissue, for example, medical stents for dilating a blood vessel, are mostly fusiform in shape. As a result, the existing conventional medical devices cannot be firmly fixed in place. In consequence, the existing conventional medical devices are likely to slide while being mounted in place or being dilated, thereby rendering it difficult to fix the medical devices to a target position. Therefore, it is necessary to provide a medical device for dilating a target tissue, with the medical device being firmly fixed in place to prevent the medical device from sliding while being mounted in place.

### SUMMARY OF THE INVENTION

In view of the aforesaid drawbacks of the prior art, it is an objective of the disclosure to provide a medical device for dilating a target tissue, with the medical device being firmly fixed in place to prevent the medical device from sliding while being mounted in place.

In order to achieve the above and other objectives, the disclosure provides a medical device for dilating a target tissue, comprising: a first lateral portion; a second lateral portion; and a middle portion connecting the first lateral portion and the second lateral portion and disposed between the first lateral portion and the second lateral portion. The medical device has a dilate mechanism for putting the first lateral portion and the second lateral portion in a first dilated state when a first criterion is met and putting the middle portion in a second dilated state when a second criterion is met. The middle portion is not in the second dilated state when the first criterion is met.

In a preferred embodiment of the disclosure, during a dilating process, the medical device meets the first criterion and then meets the second criterion.

In a preferred embodiment of the disclosure, the target tissue is a blood vessel, and the medical device is a blood vessel dilating device.

In a preferred embodiment of the disclosure, the medical device has a stent body comprising the first lateral portion, the second lateral portion and the middle portion.

In a preferred embodiment of the disclosure, the dilate mechanism allows the first lateral portion to be made of a first memory metal, the second lateral portion to be made of a second memory metal, and the middle portion to be made of a third memory metal, wherein the first lateral portion and the second lateral portion are in the first dilated state when the first criterion is met, and the middle portion is in the second dilated state when the second criterion is met, wherein the middle portion is not in the second dilated state when the first criterion is met.

In a preferred embodiment of the disclosure, the first criterion is defined for the stent body to have reached a first temperature, and the second criterion is defined for the stent body to have reached a second temperature, with the first temperature being lower than the second temperature.

In a preferred embodiment of the disclosure, the medical device has a balloon body, and the balloon body comprises the first lateral portion, the second lateral portion and the middle portion.

In a preferred embodiment of the disclosure, the dilate mechanism allows the first lateral portion to be of a first density, the second lateral portion to be of a second density, and the middle portion to be of a third density, with the third density being different from the first density and the second density.

In a preferred embodiment of the disclosure, the dilate mechanism allows the first lateral portion to be of a first thickness, the second lateral portion to be of a second thickness, and the middle portion to be of a third thickness, with the third thickness being different from the first thickness and the second thickness.

In a preferred embodiment of the disclosure, the first criterion is defined for the balloon body to be internally under a first perfusion pressure, and the second criterion is defined for the balloon body to be internally under a second perfusion pressure, with the first perfusion pressure being lower than the second perfusion pressure.

In a preferred embodiment of the disclosure, the first lateral portion has a first auxiliary member, the second lateral portion has a second auxiliary member, and the middle portion has a third auxiliary member, wherein the dilate mechanism allows the third auxiliary member to be of a greater elasticity coefficient than the first auxiliary member, and allows the third auxiliary member to be of a greater elasticity coefficient than the second auxiliary member.

In a preferred embodiment of the disclosure, the first lateral portion has a first auxiliary member, the second lateral portion has a second auxiliary member, and the middle portion has a third auxiliary member, wherein the dilate mechanism allows a third arrangement density of the third auxiliary member to be greater than a first arrangement density of the first auxiliary member, and allows the third arrangement density of the third auxiliary member to be greater than a second arrangement density of the second auxiliary member.

In a preferred embodiment of the disclosure, the first lateral portion has a first auxiliary member, the second lateral portion has a second auxiliary member, and the middle portion has a third auxiliary member, wherein the dilate mechanism allows a third extension direction of the third auxiliary member to be perpendicular to a surface extension direction of the balloon body, and allows a first extension direction of the first auxiliary member and/or a second extension direction of the second auxiliary member to be parallel to the surface extension direction.

The aforesaid aspects and other aspects of the disclosure are illustrated by non-restrictive, specific embodiments, depicted by accompanying drawings, and described below.

### BRIEF DESCRIPTION OF THE DRAWINGS

FIG. 1A is a schematic view of a medical device for dilating a target tissue according to a specific embodiment of the disclosure.
FIG. 1B is a schematic view of the medical device for dilating a target tissue according to a specific embodiment of the disclosure.
FIG. 1C is a schematic view of the medical device for dilating a target tissue according to a specific embodiment of the disclosure.
FIG. 2A is a schematic view of a medical device for dilating a target tissue according to a specific embodiment of the disclosure.
FIG. 2B is a schematic view of the medical device for dilating a target tissue according to a specific embodiment of the disclosure.
FIG. 2C is a schematic view of the medical device for dilating a target tissue according to a specific embodiment of the disclosure.
FIG. 3 is a schematic view of a medical device for dilating a target tissue according to a specific embodiment of the disclosure.
FIG. 4 is a schematic view of a medical device for dilating a target tissue according to a specific embodiment of the disclosure.
FIG. 5 is a schematic view of a medical device for dilating a target tissue according to a specific embodiment of the disclosure.

### DETAILED DESCRIPTION OF THE EMBODIMENTS

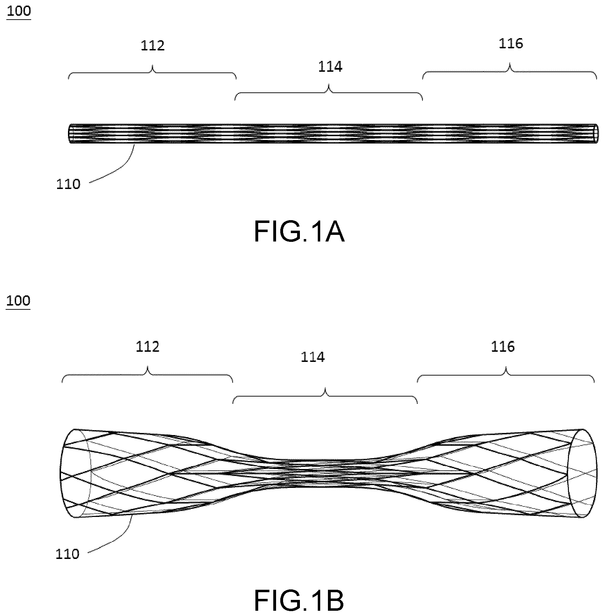

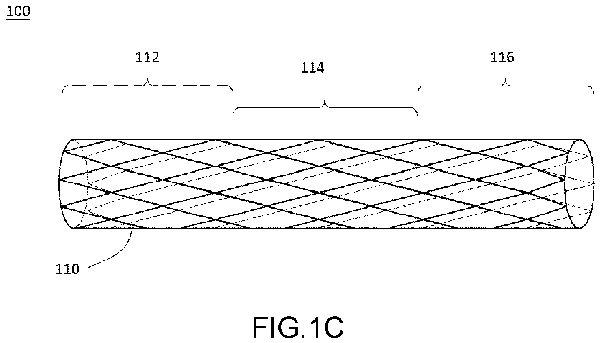

Referring to FIG. 1A through FIG. 1C, there are shown schematic views of a medical device for dilating a target tissue according to a specific embodiment of the disclosure. In the embodiment illustrated by FIG. 1A through FIG. 1C, a medical device 100 for dilating a target tissue comprises a stent body 110. (In a specific embodiment, the medical device 100 is formed by the stent body 110.) The stent body 110 comprises a first lateral portion 112, middle portion 114 and second lateral portion 116. The middle portion 114 connects the first lateral portion 112 and second lateral portion 116. The middle portion 114 is disposed between the first lateral portion 112 and second lateral portion 116. The medical device 100 has a dilate mechanism. The dilate mechanism allows the first lateral portion 112 and second lateral portion 116 to be in the first dilated state when a first criterion is met. (Referring to FIG. 1B, there is shown a schematic view of the medical device 100 in the dilated state when a first criterion is met.) The dilate mechanism further allows the middle portion 114 to be in the second dilated state when a second criterion is met. (Referring to FIG. 1C, there is shown a schematic view of the medical device 100 in the dilated state when a second criterion is met.) In the embodiment illustrated by FIG. 1B, the middle portion 114 is not in the second dilated state when the first criterion is met.

In a specific embodiment, when the stent body 110 of the medical device 100 is in the contracted state (i.e., the medical device 100 in the contracted state, as shown in FIG. 1A), a surgeon moves the medical device 100 to place it at a target position in the target tissue. Then, the surgeon allows the stent body 110 to meet the first criterion to thereby ensure that the first lateral portion 112 and second lateral portion 116 are in the first dilated state, and that the middle portion 114 is in the contracted state. At this point in time, with the stent body 110 being in a dumbbell shape, the first and second lateral portions 112, 116 in the first dilated state enable the stent body 110 to be firmly fixed to a target position in the target tissue, so as to prevent the stent body 110 in the target tissue from sliding. Then, the surgeon allows the stent body 110 to meet the second criterion to thereby ensure that the middle portion 114 is in the second dilated state and thus finish the dilate procedure. When the stent body 110 meets the second criterion, the first lateral portion 112, middle portion 114 and second lateral portion 116 are in the dilated state. According to the disclosure, the medical device for dilating a target tissue operates in a two-stage dilating mode (including a first stage characterized by a dumbbell-shaped dilated state associated with the first criterion and a second stage characterized by a fully dilated state associated with the second criterion) during a dilating process to prevent the medical device in the target tissue from being wrongly positioned as a result of its sliding within the target tissue.

In a specific embodiment, the dilate mechanism of the medical device 100 allows the first lateral portion 112 to be made of a first memory metal, the second lateral portion 116 to be made of a second memory metal, and the middle portion 114 to be made of a third memory metal. The first lateral portion 112 and second lateral portion 116 regain their original shapes (i.e., the dilated state) when the first criterion is met, such that the first lateral portion 112 and second lateral portion 116 are in the first dilated state. The middle portion 114 regains its original shape (i.e., the dilated state) when the second criterion is met, such that the middle portion 114 is in the second dilated state. The middle portion 114 cannot regain its original shape when the first criterion is met, and thus the middle portion 114 is not in the second dilated state when the first criterion is met.

In a specific embodiment, during a dilating process, the medical device 100 meets the first criterion and then meets the second criterion. In a specific embodiment, the first criterion is defined for the stent body 110 to have reached a first temperature, and the second criterion is defined for the stent body 110 to have reached a second temperature. The first temperature is lower than the second temperature. In a specific embodiment, given a perfusion liquid, the stent body 110 reaches the first temperature and then reaches the second temperature. The operation of the medical device for dilating a target tissue according to the present disclosure is not restricted to using a perfusion liquid to enable a stent body to reach a first temperature or a second temperature but may also entail passing electric current or using any other means to enable a stent body to reach a first temperature or a second temperature as needed.

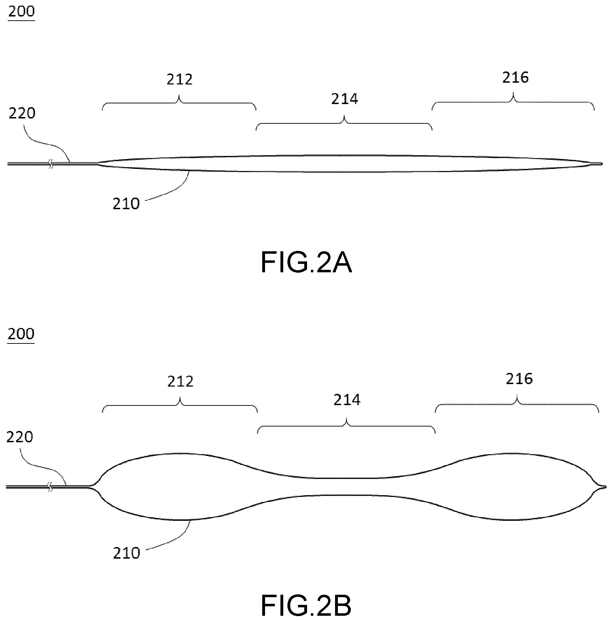

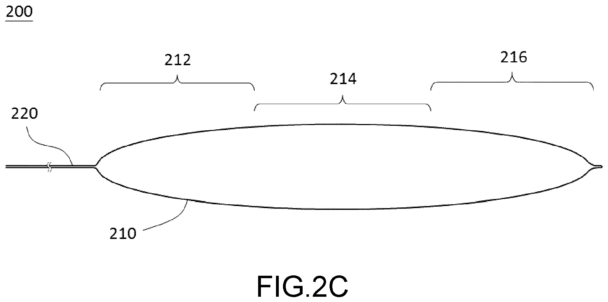

Referring to FIG. 2A through FIG. 2C, there are shown schematic views of a medical device for dilating a target tissue according to a specific embodiment of the disclosure. In the embodiment illustrated by FIG. 2A through FIG. 2C, a medical device 200 for dilating a target tissue comprises a balloon body 210 and an inflation portion 220. The balloon body 210 comprises a first lateral portion 212, middle portion 214 and second lateral portion 216. The middle portion 214 connects the first lateral portion 212 and second lateral portion 216. The middle portion 214 is disposed between the first lateral portion 212 and second lateral portion 216. The inflation portion 220 is connected to the first lateral portion 212 of the balloon body 210; thus, fluid (for example, gas or liquid) is introduced into the balloon body 210 through the inflation portion 220. In the embodiment illustrated by FIG. 2A through FIG. 2C, the medical device 200 has a dilate mechanism. The dilate mechanism allows the first lateral portion 212 and second lateral portion 216 to be in the first dilated state when the first criterion is met (i.e., the medical device 200 in the dilated state associated with the first criterion as shown in FIG. 2B). The dilate mechanism further allows the middle portion 214 to be in the second dilated state when the second criterion is met (i.e., the medical device 200 in the dilated state associated with the second criterion as shown in FIG. 2C). In the embodiment illustrated by FIG. 2B, the middle portion 214 is not in the second dilated state when the first criterion is met.

In a specific embodiment, when the balloon body 210 of the medical device 200 is in the contracted state (i.e., the medical device 200 in the contracted state as shown in FIG. 2A), the surgeon moves the medical device 200 to place it at a target position in the target tissue. Then, the surgeon allows the balloon body 210 to meet the first criterion, such that the first lateral portion 212 and second lateral portion 216 are in the first dilated state, and the middle portion 214 is in the contracted state. At this point in time, with the balloon body 210 being in a dumbbell shape, the first and second lateral portions 212, 216 in the first dilated state enable the balloon body 210 to be firmly fixed to a target position in the target tissue, so as to prevent the balloon body 210 in the target tissue from sliding. Then, the surgeon allows the balloon body 210 to meet the second criterion to thereby ensure that the middle portion 214 is in the second dilated state and thus finish the dilate procedure. When the balloon body 210 meets the second criterion, the first lateral portion 212, middle portion 214 and second lateral portion 216 are in the dilated state. According to the disclosure, the medical device for dilating a target tissue operates in a two-stage dilating mode (including a first stage characterized by a dumbbell-shaped dilated state associated with the first criterion and a second stage characterized by a fully dilated state associated with the second criterion) during a dilating process to prevent the medical device in the target tissue from being wrongly positioned as a result of its sliding within the target tissue.

In a specific embodiment, the dilate mechanism of the medical device 200 allows the first lateral portion 212 to be of a first density, the second lateral portion 216 to be of a second density, and the middle portion214 to be of a third density. The third density is different from the first density and second density. In a specific embodiment, the third density is greater than the first density and second density. The first criterion is defined for the balloon body to be internally under a first perfusion pressure, and the second criterion is defined for the balloon body to be internally under a second perfusion pressure. The first perfusion pressure is lower than the second perfusion pressure. Thus, when the balloon body 210 meets the first criterion, the first lateral portion 212 and second lateral portion 216 are in the first dilated state, and the middle portion 214 is in the contracted state. When the balloon body 210 is in the second criterion, the first lateral portion 212, middle portion 214 and second lateral portion 216 are in the dilated state. In a specific embodiment, the first density is equal to the second density. In a specific embodiment, during a dilating process, the medical device 200 meets the first criterion and then meets the second criterion.

In a specific embodiment, the dilate mechanism of the medical device 200 allows the first lateral portion 212 to be of a first thickness, the second lateral portion 216 to be of a second thickness, and the middle portion214 to be of a third thickness. The third thickness is different from the first thickness and second thickness. In a specific embodiment, the third thickness is greater than the first thickness and second thickness. The first criterion is defined for the balloon body to be internally under a first perfusion pressure, and the second criterion is defined for the balloon body to be internally under a second perfusion pressure. The first perfusion pressure is lower than the second perfusion pressure. Thus, when the balloon body 210 meets the first criterion, the first lateral portion 212 and second lateral portion 216 are in the first dilated state, and the middle portion 214 is in the contracted state. When the balloon body 210 meets the second criterion, the first lateral portion 212, middle portion 214 and second lateral portion 216 are in the dilated state. In a specific embodiment, the first thickness is equal to the second thickness. In a specific embodiment, during a dilating process, the medical device 200 meets the first criterion and then meets the second criterion.

In a specific embodiment, according to the disclosure, the medical device for dilating a target tissue is a blood vessel dilating device for use in dilating blood vessels (i.e., arteries and veins) in a target tissue. According to the disclosure, the medical device for dilating a target tissue is not restricted to a blood vessel dilating device. The application of the medical device of the disclosure is not restricted to blood vessels; instead, the medical device of the disclosure may also be used to dilate any tissue of the human body as needed. According to the disclosure, the medical device for dilating a target tissue is of various sizes, depending on the size of the target tissue.

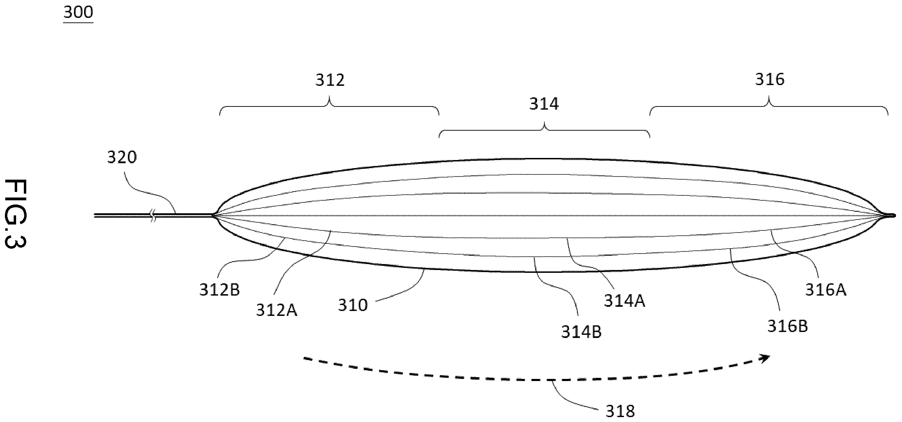

Referring to FIG. 3, there is shown a schematic view of a medical device for dilating a target tissue according to a specific embodiment of the disclosure. In the embodiment illustrated by FIG. 3, a medical device 300 for dilating a target tissue comprises a balloon body 310 and an inflation portion 320. The balloon body 310 comprises a first lateral portion 312, middle portion 314 and second lateral portion 316. The middle portion 314 connects the first lateral portion 312 and second lateral portion 316. The middle portion 314 is disposed between the first lateral portion 312 and second lateral portion 316. The inflation portion 320 is connected to the first lateral portion 312 of the balloon body 310; thus, fluid (for example, gas or liquid) is introduced into the balloon body 310 through the inflation portion 320. The medical device 300 has a dilate mechanism. The dilate mechanism allows the first lateral portion 312 and second lateral portion 316 to be in the first dilated state when the first criterion is met (i.e., the medical device 200 in the dilated state associated with the first criterion as shown in FIG. 2B). The dilate mechanism further allows the middle portion 314 to be in the second dilated state when the second criterion is met (i.e., the medical device 200 in the dilated state associated with the second criterion as shown in FIG. 2C). The middle portion 314 is not in the second dilated state when the first criterion is met.

In the embodiment illustrated by FIG. 3, the first lateral portion 312 has first auxiliary members 312A, 312B, the second lateral portion 316 has second auxiliary members 316A, 316B, and the middle portion 314 has third auxiliary members 314A, 314B. The first auxiliary members 312A, 312B have a first elasticity coefficient, the second auxiliary members 316A, 316B have a second elasticity coefficient, and the third auxiliary members 314A, 314B have a third elasticity coefficient. The dilate mechanism of the medical device 300 allows the third elasticity coefficient of the third auxiliary members 314A, 314B to be greater than the first elasticity coefficient of the first auxiliary members 312A, 312B, and allows the third elasticity coefficient of the third auxiliary members 314A, 314B to be greater than the second elasticity coefficient of the second auxiliary members 316A, 316B. Thus, during a dilating process, the medical device 300 meets the first criterion and then meets the second criterion. In a specific embodiment, the first auxiliary members 312A, 312B, second auxiliary members 316A, 316B, and third auxiliary members 314A, 314B are fabrics. In a specific embodiment, the first auxiliary members 312A, 312B are connected to the third auxiliary members 314A, 314B, respectively, and the second auxiliary members 316A, 316B are connected to the third auxiliary members 314A, 314B, respectively. In another specific embodiment, the first auxiliary members 312A, 312B are not connected to the third auxiliary members 314A, 314B, and the second auxiliary members 316A, 316B are not connected to the third auxiliary members 314A, 314B. The extension direction of the first auxiliary members 312A, 312B is parallel to the surface extension direction 318 of the balloon body 310 as needed or perpendicular to the surface extension direction 318 of the balloon body 310 as needed, but the disclosure is not restricted thereto. The extension direction of the second auxiliary member 316A, 316B is parallel to the surface extension direction 318 of the balloon body 310 as needed or perpendicular to the surface extension direction 318 of the balloon body 310 as needed, but the disclosure is not restricted thereto. The extension direction of the third auxiliary members 314A, 314B is parallel to the surface extension direction 318 of the balloon body 310 as needed or perpendicular to the surface extension direction 318 of the balloon body 310 as needed, but the disclosure is not restricted thereto.

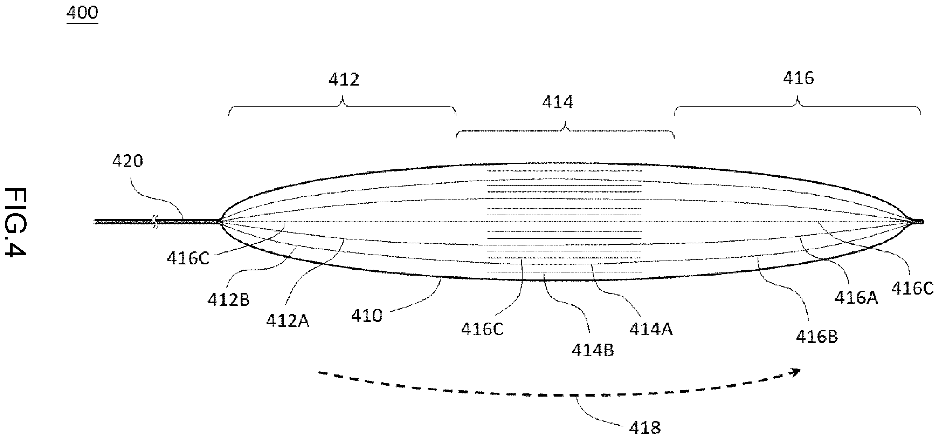

Referring to FIG. 4, there is shown a schematic view of a medical device for dilating a target tissue according to a specific embodiment of the disclosure. In the embodiment illustrated by FIG. 4, a medical device 400 for dilating a target tissue comprises a balloon body 410 and an inflation portion 420. The balloon body 410 comprises a first lateral portion 412, middle portion 414 and second lateral portion 416. The middle portion 414 connects the first lateral portion 412 and second lateral portion 416. The middle portion 414 is disposed between the first lateral portion 412 and second lateral portion 416. The inflation portion 420 is connected to the first lateral portion 412 of the balloon body 410; thus, fluid (for example, gas or liquid) is introduced into the balloon body 410 through the inflation portion 420. The medical device 400 has a dilate mechanism. The dilate mechanism allows the first lateral portion 412 and second lateral portion 416 to be in the first dilated state when the first criterion is met (i.e., the medical device 200 in the dilated state associated with the first criterion as shown in FIG. 2B). The dilate mechanism further allows the middle portion 414 to be in the second dilated state when the second criterion is met (i.e., the medical device 200 in the dilated state associated with the second criterion as shown in FIG. 2C). The middle portion 414 is not in the second dilated state when the first criterion is met.

In the embodiment illustrated by FIG. 4, the first lateral portion 412 has first auxiliary members 412A, 412B, 412C, the second lateral portion 416 has second auxiliary members 416A, 416B, 416C, and the middle portion 414 has third auxiliary members 414A, 414B, 414C. The first auxiliary members 412A, 412B, 412C have a first arrangement density therebetween. The second auxiliary members 416A, 416B, 416C have a second arrangement density therebetween. The third auxiliary members 414A, 414B, 414C have a third arrangement density therebetween. The dilate mechanism of the medical device 400 allows the third arrangement density of the third auxiliary members 414A, 414B, 414C to be greater than the first arrangement density of the first auxiliary members 412A, 412B, 412C, and allows the third arrangement density of the third auxiliary members 414A, 414B, 414C to be greater than the second arrangement density of the second auxiliary members 416A, 416B, 416C. In a specific embodiment, the first auxiliary members 412A, 412B, 412C, second auxiliary members 416A, 416B, 416C, and third auxiliary members 414A, 414B, 414C are fabrics. In a specific embodiment, the first auxiliary member is connected to at least a portion of the third auxiliary member, and the second auxiliary member is connected to at least a portion of the third auxiliary member. In another specific embodiment, the first auxiliary member is not connected to the third auxiliary member, and the second auxiliary member is not connected to the third auxiliary member. The extension direction of the first auxiliary members 412A, 412B, 412C is parallel to the surface extension direction 418 of the balloon body 410 as needed or perpendicular to the surface extension direction 418 of the balloon body 410 as needed, but the disclosure is not limited thereto. The extension direction of the second auxiliary members 416A, 416B, 416C is parallel to the surface extension direction 418 of the balloon body 410 as needed or perpendicular to the surface extension direction 418 of the balloon body 410 as needed, but the disclosure is not limited thereto. The extension direction of the third auxiliary members 414A, 414B, 414C is parallel to the surface extension direction 418 of the balloon body 410 as needed or perpendicular to the surface extension direction 418 of the balloon body 410 as needed, but the disclosure is not limited thereto.

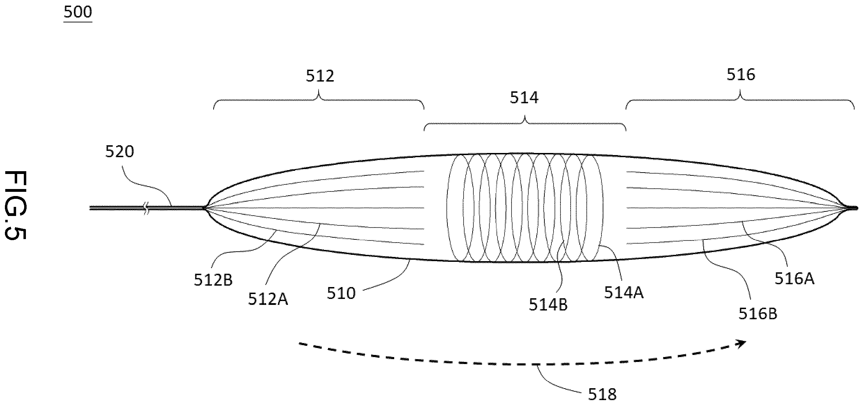

Referring to FIG. 5, there is shown a schematic view of a medical device for dilating a target tissue according to a specific embodiment of the disclosure. In the embodiment illustrated by FIG. 5, a medical device 500 for dilating a target tissue comprises a balloon body 510 and an inflation portion 520. The balloon body 510 comprises a first lateral portion 512, middle portion 514 and second lateral portion 516. The middle portion 514 connects the first lateral portion 512 and second lateral portion 516. The middle portion 514 is disposed between the first lateral portion 512 and second lateral portion 516. The inflation portion 520 is connected to the first lateral portion 512 of the balloon body 510; thus, fluid (for example, gas or liquid) is introduced into the balloon body 510 through the inflation portion 520. The medical device 500 has a dilate mechanism. The dilate mechanism allows the first lateral portion 512 and second lateral portion 516 to be in the first dilated state when the first criterion is met (i.e., the medical device 200 in the dilated state associated with the first criterion as shown in FIG. 2B). The dilate mechanism further allows the middle portion 514 to be in the second dilated state when the second criterion is met (i.e., the medical device 200 in the dilated state associated with the second criterion as shown in FIG. 2C). The middle portion 514 is not in the second dilated state when the first criterion is met.

In the embodiment illustrated by FIG. 5, the first lateral portion 512 has first auxiliary members 512A, 512B, the second lateral portion 516 has second auxiliary members 516A, 516B, and the middle portion 514 has third auxiliary members 514A, 514B. The first auxiliary members 512A, 512B have a first extension direction. The second auxiliary members 516A, 516B have a second extension direction. The third auxiliary members 514A, 514B have a third extension direction. The dilate mechanism of the medical device 500 allows the third extension direction of the third auxiliary members 514A, 514B to be perpendicular to the surface extension direction 518 of the balloon body 510, and allows the first extension direction of the first auxiliary members 512A, 512B and/or the second extension direction of the second auxiliary members 516A, 516B to be parallel to the surface extension direction 518 of the balloon body 510. In a specific embodiment, the first auxiliary members 512A, 512B, second auxiliary members 516A, 516B, and third auxiliary members 514A, 514B are fabrics. In a specific embodiment, the first auxiliary members are connected to the third auxiliary members, and the second auxiliary members are connected to the third auxiliary members. In another specific embodiment, the first auxiliary members are not connected to the third auxiliary members, and the second auxiliary members are not connected to the third auxiliary members.

The medical device for dilating a target tissue of the disclosure is depicted by drawings and described above. Specific embodiments of the disclosure merely serve illustrative purposes. Various changes made to the specific embodiments without departing from the spirit and claims of the disclosure must be deemed falling within the scope of the claims of the disclosure. Accordingly, the spirit and scope of the disclosure should be defined by the appended claims, and the specific embodiments described herein are not restrictive of the disclosure.

## Claims

1. A medical device for dilating a target tissue, comprising:
a first lateral portion;
a second lateral portion; and
a middle portion connecting the first lateral portion and the second lateral portion and disposed between the first lateral portion and the second lateral portion,
wherein the medical device has a dilate mechanism enabling the first lateral portion and the second lateral portion to be in a first dilated state when a first criterion is met and
enabling the middle portion to be in a second dilated state when a second criterion is met,
wherein the middle portion is not in the second dilated state when the first criterion is met.

2. The medical device of claim 1, wherein, during a dilating process, the medical device meets the first criterion and then meets the second criterion.

3. The medical device of claim 1, wherein the target tissue is a blood vessel, and the medical device is a blood vessel dilating device.

4. The medical device of claim 1, wherein the medical device has a stent body comprising the first lateral portion, the second lateral portion and the middle portion.

5. The medical device of claim 4, wherein the dilate mechanism allows the first lateral portion to be made of a first memory metal, the second lateral portion to be made of a second memory metal, and the middle portion to be made of a third memory metal, wherein the first lateral portion and the second lateral portion are in the first dilated state when the first criterion is met, and the middle portion is in the second dilated state when the second criterion is met, wherein the middle portion is not in the second dilated state when the first criterion is met.

6. The medical device of claim 5, wherein the first criterion is defined for the stent body to have reached a first temperature, and the second criterion is defined for the stent body to have reached a second temperature, with the first temperature being lower than the second temperature.

7. The medical device of claim 1, wherein the medical device has a balloon body, and the balloon body comprises the first lateral portion, the second lateral portion and the middle portion.

8. The medical device of claim 7, wherein the dilate mechanism allows the first lateral portion to be of a first density, the second lateral portion to be of a second density, and the middle portion to be of a third density, with the third density being different from the first density and the second density.

9. The medical device of claim 7, wherein the dilate mechanism allows the first lateral portion to be of a first thickness, the second lateral portion to be of a second thickness, and the middle portion to be of a third thickness, with the third thickness being different from the first thickness and the second thickness.

10. The medical device of claim 7, wherein the first criterion is defined for the balloon body to be internally under a first perfusion pressure, and the second criterion is defined for the balloon body to be internally under a second perfusion pressure, with the first perfusion pressure being lower than the second perfusion pressure.

11. The medical device of claim 7, wherein the first lateral portion has a first auxiliary member, the second lateral portion has a second auxiliary member, and the middle portion has a third auxiliary member, wherein the dilate mechanism allows the third auxiliary member to be of a greater elasticity coefficient than the first auxiliary member, and allows the third auxiliary member to be of a greater elasticity coefficient than the second auxiliary member.

12. The medical device of claim 7, wherein the first lateral portion has a first auxiliary member, the second lateral portion has a second auxiliary member, and the middle portion has a third auxiliary member, wherein the dilate mechanism allows a third arrangement density of the third auxiliary member to be greater than a first arrangement density of the first auxiliary member, and allows the third arrangement density of the third auxiliary member to be greater than a second arrangement density of the second auxiliary member.

13. The medical device of claim 7, wherein the first lateral portion has a first auxiliary member, the second lateral portion has a second auxiliary member, and the middle portion has a third auxiliary member, wherein the dilate mechanism allows a third extension direction of the third auxiliary member to be perpendicular to a surface extension direction of the balloon body, and allows a first extension direction of the first auxiliary member and/or a second extension direction of the second auxiliary member to be parallel to the surface extension direction.
